# EUROPEAN PATENT APPLICATION

(11) **EP 2 597 583 A1**
(43) Date of publication of application: **29.05.2013**
(21) Application number: 12187101.6
(22) Date of filing: 03.10.2012
(51) Int. Cl.: G06F 19/00, G06Q 50/22

(54) **System and method for scheduling home visits for physician review**

(30) Priority: 28.11.2011 US 201113305534
(71) Applicant: Censeo Health LLC, Dallas, TX 75244 (US)
(72) Inventor: Piot, Jon C., Dallas, TX Texas 75244 (US)
(74) Representative: Jackson, Richard Eric

(57) **Abstract**

A computer implemented method for scheduling physician visits for medical care validation that involves a processor selecting members to be visited based on the proximity of the selected member to a previously scheduled member. From these selections, a route is prepared for the physician that minimizes the travel time between member visits.

## Description

### CROSS-REFERENCE TO RELATED APPLICATIONS

The present application is related to commonly assigned U.S. Patent Application Serial No. 13/305,503 filed on November 28, 2011 entitled "SYSTEM AND METHOD FOR ANALYZING AUDIT RISK OF CLAIMS-BASED SUBMISSIONS FOR MEDICARE ADVANTAGE RISK ADJUSTMENT," the disclosure of which is hereby incorporated by referenced herein in its entirety.

### TECHNICAL FIELD

The present disclosure generally relates to validation of medical care that has been provided to members of a health plan. More specifically, the present disclosure relates to scheduling physician visits to a plurality of members so that the physicians can prepare charts that support the medical care received by members.

### BACKGROUND OF THE INVENTION

Provision of modem day medical care often involves an entity other than the patient paying at least some of the cost of the medical care. For example, some of a patient's medical costs may be paid by insurance companies and/or government institutions. In the case of the United States, Medicare Advantage Plans (herein "the Plans") are run by private insurance companies that accept the underwriting and marketing responsibilities for Medicare members. The payment methodology which the Centers for Medicare and Medicaid Services, (CMS) has established, allows a Plan to be paid different amounts for different members of the Plan based on a set of identified diagnoses and treatments of those members.

To ensure disbursements made by CMS to insurance companies are proper, Medicare audits a certain amount of an insurance company's reimbursed claims. The audit usually involves checking whether chart records support diagnoses and treatments. If the audit reveals that minimum standards have not been met for the audited cases, the relevant insurance company may be fined. The amount of the fine is determined by extrapolating the percent of cases not meeting the minimum standards to the total reimbursement made to the company over a period. Therefore, it is important that insurance companies meet the minimum standards when audited. Thus, systems and methods, such as those disclosed in the above-referenced patent application entitled: "SYSTEM AND METHOD FOR ANALYZING AUDIT RISK OF CLAIMS-BASED SUBMISSIONS FOR MEDICARE ADVANTAGE RISK ADJUSTMENT" seek to identify cases that are likely not to meet the minimum standards and take corrective action to meet those standards prior to an audit. Part of the corrective action involves a physician visiting the members involved in these cases to validate diagnoses and treatments by properly preparing medical charts for the members in question. The word "member" as used herein means the patient or the person whose medical care is covered by the Plans.

A physician is required to do the validation. Usually, the validations have to be done at the residence of the member. Thus, arrangements have to be made for the physician to visit with the member at the member's home. There are challenges to doing this efficiently. First, a time convenient to the member has to be scheduled for the physician's visit. Second, the physician needs to be on time. Third, the physician has to see a minimum amount of patients per day in order for the whole operation to be efficient.

### BRIEF SUMMARY OF THE INVENTION

Embodiments of the present disclosure are directed to a computer implemented method for scheduling physician visits for medical care validation. The physician's schedule is prepared by identifying a next scheduled visit location based on the location of a previously scheduled visit. For example, one embodiment of the invention involves a processor presenting, to a scheduler, contact information of a first member. From this information, the scheduler may contact the member to try and set a first appointment for the physician. If the member agrees to the appointment, the scheduler gives instructions to the processor to schedule the first appointment for the physician to meet with the first member. Based on these instructions, the processor sets the first appointment. The processor then selects, from a database, a second member for a prospective second appointment. The second member is selected from members not yet contacted by the scheduler. The second member is selected based on the second member being located closer to the first member than other members not yet contacted by the scheduler. The processor then presents to the scheduler, contact information of the second member for the scheduler to contact the member to set a second appointment for the physician. If the scheduler receives agreement of the member, the processor is instructed to schedule a second appointment for the physician to meet with the second member. Based on this, the processor schedules the second appointment.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE DRAWINGS

For a more complete understanding of the present invention, reference is now made to the following descriptions taken in conjunction with the accompanying drawing, in which:

FIGURE 1 is a method of scheduling physician visits according to one embodiment of the invention; and

FIGURE 2 is a system for scheduling physician visits according to one embodiment of the invention;

### DETAILED DESCRIPTION OF THE INVENTION

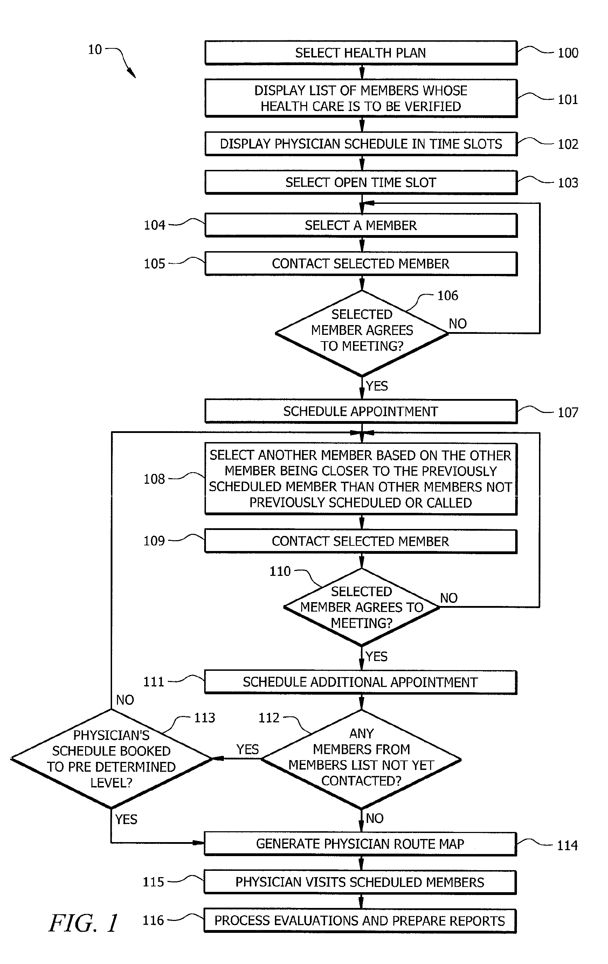

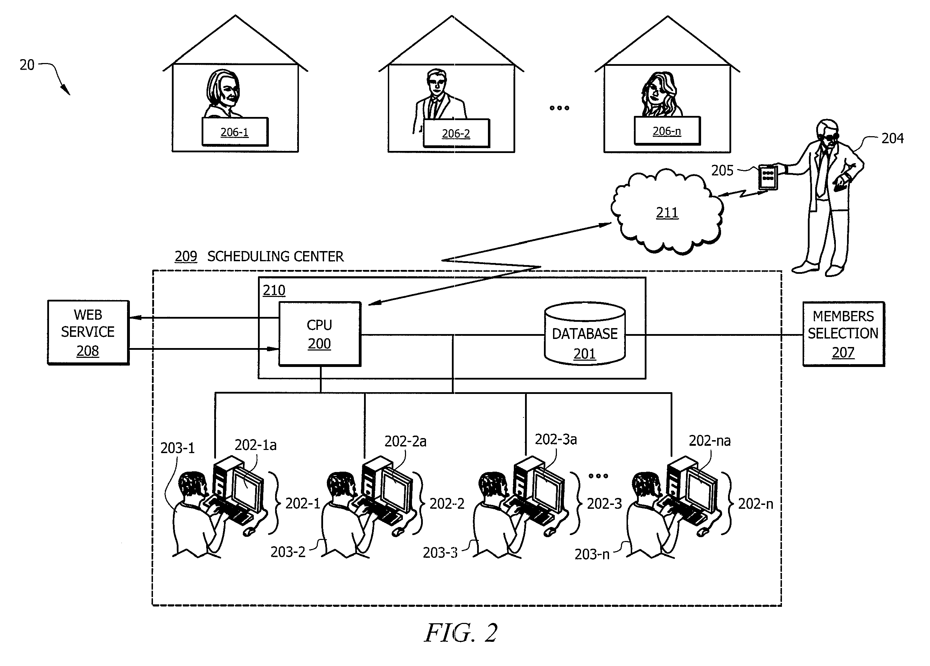

FIGURE 1 shows method 10 for scheduling physician visits according to one embodiment of the invention. FIGURE 2 shows a system that may be used to implement methods according to embodiments of the invention such as method 10. System 20 includes a central processing unit ("CPU" or "processor") 200 and database 201. System 20 also includes workstations 202-1 - 202-n, which are communicatively connected to processor 200 and database 201. Workstations 202-1 - 202-n include electronic displays 202-1 a - 202-na and are operated by schedulers 203-1 - 203-n. Database 201 stores a list of all the members that physicians need to visit for validation of their diagnoses and treatments. This member list may be produced from a member selection process, such as, member selection module 207. Member selection module 207 involves the use of algorithms to select members based on a review of claims information and different criteria pertaining to the member's age, medical condition, prior diagnoses etc. The algorithm logic processes select a subset of the total membership that presents the highest return on investment (ROI) for the health plan to seek validation. Further details of the member selection process are described in the above-referenced patent application entitled: "SYSTEM AND METHOD FOR ANALYZING AUDIT RISK OF CLAIMS-BASED SUBMISSIONS FOR MEDICARE ADVANTAGE RISK ADJUSTMENT."

With reference to FIGURES 1 and 2, method 10 includes step 100, which involves, for example, scheduler 203-1 using workstation 202-1 to select a particular client or health care plan for which scheduler 203-1 is going to schedule physician visits. Based on the selection of the client in step 100, processor 200 presents the applicable evaluation form and voice script for that health plan. That is, the evaluation forms and scripts are client specific. For example, a client's evaluation form may require an answer to the question: "Do you smoke?" Other clients' evaluation forms may not require this question. Further, the scheduler's script will change, for example, to identify the client for whom the call is being made. All this information may be presented to scheduler 203-1 by processor 200 at workstation 202-1.

At step 101, scheduler 203-1 using workstation 202-1, requests that processor 200 recalls information regarding a plurality of members, such as members 206-1 to 206-n, to be scheduled so that their medical diagnosis and treatment can be validated. Processor 200 recalls this information from database 201 and presents it in a list on display 202-1 a. In embodiments, the member list presented is based on members residing in a particular geographical area such that a physician, such as physician 204, could visit several of these members in one day. Scheduler 203-1 will then request a display of the physician's schedule for a particular day at step 102. The physicians working day is divided into time slots. In some embodiments, each time slot is one hour long. The scheduler's objective is to fill each time slot with an appointment to visit a member. When the scheduler opens the physician's schedule, the time slots may be fully booked, partially booked or have no bookings at all. For easy identification, the time slots may be color coded to indicate their status. For example, green may indicate a time slot is free, red may indicate the time slot has already been booked and orange may indicate that a tentative appointment has been made.

At step 103, the scheduler selects an open timeslot displayed on screen 202-1a. If this is the first time slot for the day, processor 20 will then select, at step 104, a member to be visited by the physician. The member may be selected based on that member's location being closer to the physician's base location from which the physician's route will be started (e.g. home or hotel). Alternatively the member for the first time slot could be selected randomly from the member list. Processor 200 will then display on display 202-1 a, the selected member's information, which includes the member's contact information such as phone number and address. At step 105, the scheduler will place a telephone call to the selected member to make an appointment for the physician to meet with the selected member during the first time slot. At step 106, it is determined whether the first member agrees to the proposed meeting. If the member does not agree to the proposed meeting, at step 104, the scheduler moves on to selecting another member to call. If the member agrees to the proposed meeting, at step 107, the scheduler instructs the processor to schedule the first appointment for the physician to meet with the first member. On receipt of these instructions, the processor schedules the first appointment and, if the color coding mentioned above is used, display 201-1 a, under control of processor 200, displays the time slot in red.

In response to the booking of the first slot, at step 108, processor 200 selects, from database 201, a second member for the scheduler to call and try to make the physician's second appointment of the day. The second member is selected from members of the plurality of members of the member list that have not been previously scheduled or contacted by the scheduler. The second member is selected based on the second member being located closer to the previously scheduled member (first member) than other members of the plurality of members not yet scheduled or contacted by the scheduler. It should be noted that if a member has declined an opportunity for a physician visit, the scheduler will save this information to database 201 so that processor 200 can read this information and not select these members that have previously declined visits.

Determining the distance of one member location from another member location (to establish which member is closest to a previously scheduled member) can be achieved in different ways. In one embodiment, addresses are used to determine the distance of one member's location to another member's location. In embodiments, distance is measured by road, which is derived from the addresses of the relevant members. For example, processor 200 may run an application that uses map data to calculate distances between member locations based on the addresses of the locations. In some embodiments, the addresses may be submitted by processor 200 to a web service, such as web service 208, which calculates the distances and returns that information to processor 200. In some embodiments, processor 200 supplies street addresses of member locations to web service 208, which returns data of the locations' latitude and longitude, to processor 200. Processor 200 uses this information to calculate distance from one address to another. In embodiments of the invention, instead of calculating the distance by road, the distance may be calculated "as the crow flies" *i.e.* in a straight line on a map. Once the second member is selected based on the proximity to the first member location, processor 200 directs the display of the information of the second member on electronic display 202-1a.

At step 109, the scheduler will call the second selected member and try to make an appointment for the physician to meet with the second selected member during the second time slot. At step 110, it is determined whether the member agrees to the proposed meeting. If the member does not agree to the proposed meeting, the scheduler indicates this to system 210. The scheduler has the option to enter into database 201, information derived from the calls with the members. For example, the scheduler can change the status of members to indicate any of the following: (1) scheduled for appointment, (2) need to call back, (3) left message (4) declined etc. Based on scheduler 203-1 informing system 210 that the member does not agree to the proposed meeting, processor 200 moves on to selecting another member to call at step 108 (*i.e.* selecting the member located the next shortest distance away from the first member). It should be noted that if the member is available at a different time and/or day, and that time/day is shown to be open, scheduler 203-1 may book that member for that day and time. If the member agrees to the proposed meeting, at step 111, scheduler 203-1 instructs the processor to schedule the second appointment for the physician to meet with the second member. On receipt of these instructions, the processor schedules the second appointment and, if the color coding mentioned above is used, display 201-1 a, under control of processor 200, displays the booked time slot in red.

Step 112 determines whether any members from the member list have not yet been contacted. If there are members that have not been contacted, then method 10 proceeds to step 113. At step 113, it is determined whether the physician's schedule has been booked to a predetermined level. If the predetermined level has not been met, then method 10 proceeds to step 108 which selects another member as being the closest unscheduled member that has not been contacted. Steps 108 - 112 are repeated until the predetermined level is achieved or until all the members on the list have been contacted. Once the predetermined booking level has been achieved or all members on the list have been contacted, then method 10 proceeds to step 114.

At step 114, method 10 generates a route for the physician for the particular day being worked on. Developing and generating the physician's route so that the next appointment is based on the location of the appointment immediately preceding it, as described herein, minimizes the drive time between the several stops along the physician's route. Thus, driving time is reduced from the physician's home location or hotel at the beginning of the day to the first member visit and between member visits throughout the day. This also maximizes the chance of the physician being on time for appointments. The route may be presented in a text or graphical format or combinations thereof. The route may be stored in database 201, which processor 200 accesses to generate the route map. Processor 200 may email a PDF of the route map to the physician or send it to an electronic PDA - iPad, iPhone, such as mobile communication device 205. From any of these devices, the physician can access his or her schedule for a particular day. The physician may utilize the mapping software of the particular PDA to retrieve and view the route map.

Armed with the tool of a route map generated to minimize drive time, at step 115, the physician will visit the scheduled members and perform the required validations and evaluations. Once the physician has completed the validations and evaluations, the results are uploaded to database 201 by the physician. At step 116, the physician's evaluations are processed. Reports are prepared and return on investment calculations for the health plan are made, which are then converted and sent electronically to the health plan itself.

In one embodiment, software instructions/routines, such as the software instructions for scheduling physician visits (*e.g.*, steps of method 10 (FIGURE 1) performed by processor 200) are implemented as a computer program product that provides at least a portion of the software instructions for an exemplary embodiment of the present invention. The computer program product can be installed by any suitable software installation procedure, as is well known in the art. In another embodiment, at least a portion of the software instructions may also be downloaded over a cable, communication and/or wireless connection.

All or a portion of the software instructions and/or data (*e.g.*, data stored in database 201) may be communicated (*e.g.*, across network 211 and/or within a given computer device) as signals propagating on a carrier or propagation medium. As used herein, a computer-readable storage medium refers to a tangible storage medium, such as a hard disk, ROM (DVD-ROM's, CD-ROM's), RAM, flash memory device, magnetic memory device (diskettes, tapes, etc.), and is not intended to refer merely to a propagating signal. As described herein, various processes (*e.g.,* steps of method 10 (FIGURE 1) performed by processor 200) may be implemented as computer-executable software instructions (or applications) that are stored to a computer-readable storage medium (*e.g.*, hard disk, ROM, RAM, flash memory device, magnetic memory device, etc.) that when executing on a processor-based device (*e.g.*, processor 200) performs the corresponding operations described herein.

Various elements of embodiments of the present invention may be implemented as computer-executable software instructions/applications stored to a computer-readable storage medium (*e.g.*, hard disk, ROM, RAM, flash memory device, magnetic memory device, etc.) that when executing on a processor-based device (*e.g.*, processor 200) provides the corresponding functionality described herein for such element.

Many of the elements described herein, when implemented via computer-executable instructions, are in essence the software code defining the operations thereof. For instance, the above-described steps of method 10 (FIGURE 1) performed by processor 200 may comprise computer-executable software code that is stored to a computer-readable storage medium and is executed by a processor-based computing device (*e.g.*, processor 200) for performing the corresponding operations described herein. Further, the various operations described herein, such as those operations described with reference to the exemplary flow of (*e.g.*, steps of method 10 (FIGURE 1) performed by processor 200), as well as other operations described herein may be performed by computer-executable software code stored to a computer-readable storage medium and executing on a processor-based computing device. The executable instructions or software code may be obtained, for example, from a computer-readable storage medium or "storage device" (*e.g.*, a hard drive media, optical media, EPROM, EEPROM, tape media, cartridge media, flash memory, ROM, memory stick, and/or the like). In certain embodiments, a CPU of a computing system or device may execute the various logical instructions according to embodiments of the present invention. For example, CPU 200 and/or workstations 202-1 - 202-n may execute machine-level instructions according to the exemplary operational flow described above in conjunction with processes described herein (*e.g.,* steps of method 10 (FIGURE 1) performed by processor 200). It shall be appreciated that the present invention is not limited to the architecture of the computing system or device on which the various elements are implemented, such as any particular architecture of system 210. The various illustrative logical blocks, modules, and circuits described in connection with the disclosure herein may be implemented or performed with a general-purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein, as examples. A general-purpose processor may be a microprocessor, but in the alternative, the processor may be any conventional processor, controller, microcontroller, or state machine. A processor may also be implemented as a combination of computing devices, *e.g.*, a combination of a DSP and a microprocessor, a plurality of microprocessors, one or more microprocessors in conjunction with a DSP core, or any other such configuration.

Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

## Claims

1. A computer implemented method for scheduling physician visits, to a plurality of members, for medical care validation, said method comprising:
(a) presenting to a scheduler, by a processor via an electronic display, contact information of a first member;
(b) receiving, by said processor from said scheduler, instructions to schedule a first appointment for a physician to meet with said first member;
(c) scheduling, by said processor, said first appointment as a function of said receiving of instructions to schedule said first appointment;
(d) selecting, by said processor from a database, an additional member for a prospective additional appointment, said selecting initiated as a result of said scheduling of said first appointment, said additional member selected from members of said plurality of members that have not been contacted by said scheduler, said additional member selected based on said additional member being located closer to said first member than other members that have not been contacted by said scheduler;
(e) presenting to said scheduler, by said processor via said electronic display, contact information of said additional member;
(f) receiving, by said processor from said scheduler, instructions to schedule an additional appointment for said physician to meet with said additional member; and
(g) scheduling, by said processor, said additional appointment as a function of said receiving of instructions to schedule said additional appointment.

2. The computer implemented method of claim 1 further comprising:
repeating steps (d) - (g), wherein, in said repeating, said first member and said first appointment of said steps (d) - (g) are defined as a last additional member and a last appointment, respectively, of prior performances of steps (d) - (g).

3. The computer implemented method of claim 2 wherein said repeating takes place until all of said plurality of members have been contacted or said physician's schedule has been booked to a predetermined level.

4. The computer implemented method of claim 3 further comprising:
preparing a route map for said physician based on said scheduled appointments.

5. The computer implemented method of claim 4 further comprising:
sending said route map to said physician's mobile communication device.

6. The computer implemented method of claim 1 wherein said additional member's closeness to said first member is based on distances between locations by road.

7. The computer implemented method of claim 1 wherein said additional member's closeness to said first member is based on distances between locations by a straight line on a map.

8. A computer program product for scheduling physician visits to a plurality of members for medical care validation, said computer program product having a computer readable medium with computer program logic recorded thereon, said computer program product comprising:
code for presenting contact information of a first member;
code for receiving instructions to schedule a first appointment for a physician to meet with said first member;
code for scheduling said first appointment as a function of receiving instructions to schedule said first appointment;
code for selecting a second member for a prospective second appointment, said selecting initiated as a result of scheduling of said first appointment, said second member selected from members of said plurality of members that have not been contacted by said scheduler, said second member selected based on said second member being located closer to said first member than other members that have not been contacted by said scheduler; and
code for presenting contact information of said second member.

9. The computer program product of claim 8 further comprising:
code for receiving instructions to schedule a second appointment for said physician to meet with said second member; and
code for scheduling said second appointment as a function of receiving instructions to schedule said second appointment.

10. The computer program product of claim 9 further comprising:
code for preparing a route map for said physician based on said scheduled appointments.

11. The computer program product of claim 9 further comprising:
code for sending said route map to said physician's mobile communication device.

12. A system for scheduling visits to a plurality of members, for medical care validation, said method comprising:
an electronic display adapted to display, to a scheduler, information pertaining to a first member;
a database including information for said plurality of members;
a processor adapted to schedule said first member based on instructions from said scheduler, said processor further adapted to select and present, to said scheduler, an additional member as a function of said first member being scheduled, said additional member selected based on said additional member being closer to said first member than other members of said plurality of members that have not been contacted by said scheduler.

13. The system of claim 12 wherein said processor is further adapted to receive instructions to schedule an additional appointment for said physician to meet with said additional member and to schedule said additional appointment as a function of receiving said instructions to schedule said additional appointment.

14. The system of claim 13 wherein said processor is further adapted to prepare a route map for said physician based on said scheduled appointments.

15. The system of claim 14 wherein said processor is further adapted to send said route map to said physician's mobile communication device.

16. A computer implemented method for preparing a route for a service provider to visit a plurality of locations, said method comprising:
(a) presenting, under control of a processor via an electronic display, to a scheduler, a first location that potentially will be visited in said route;
(b) receiving, by said processor, instructions from said scheduler that said first location is to be visited in said route;
(c) scheduling, by said processor, said first location as one to be visited in said route, said scheduling as a function of receiving said instructions regarding said first location;
(d) selecting and presenting, to said scheduler, an additional location that potentially will be a next location to be visited after said first location in said route, said selection initiated as a result of said scheduling of said first location, said additional location selected because it is the closest unscheduled one, of said plurality of locations, to said first location;
(e) receiving, by said processor, instructions from said scheduler that said additional location is to be visited in said route;
(f) based on said receiving of said instructions regarding said additional location, scheduling, by said processor, said additional location as the next location to be visited after said first location in said route.

17. The computer implemented method of claim 16 wherein said plurality of locations are residential locations of members of a health plan and said service provider is a physician that performs health care validations.

18. The computer implemented method of claim 17 further comprising:
repeating steps (d) - (f), wherein said first location in step (d) is considered a last scheduled location from previous performances of step (d).

19. The computer implemented method of claim 18 wherein said repeating takes place until all of said plurality of locations have been scheduled or said physician's schedule has been booked to a predetermined level.

20. The computer implemented method of claim 19 further comprising:
preparing, by said processor, a map of said route for said physician.

21. The computer implemented method of claim 20 further comprising:
transmitting said map of said route to said physician's mobile communication device.

22. The computer implemented method of claim 16 wherein said additional member's closeness to said first member is based on distances by road.

23. The computer implemented method of claim 16 wherein said additional member's closeness to said first member is based on distances by a straight line on a map.
